# EUROPEAN PATENT SPECIFICATION

(11) **EP 3 763 328 B1**
(45) Date of publication and mention of the grant of the patent: **21.08.2024**
(21) Application number: 19184993.4
(22) Date of filing: 08.07.2019
(51) Int. Cl.: A61F 2/24, A61M 25/01

(54) **A MEDICAL ARRANGEMENT FOR INTRODUCING AN OBJECT INTO AN ANATOMICAL TARGET POSITION**
MEDIZINISCHE ANORDNUNG ZUR EINFÜHRUNG EINES OBJEKTS IN EINE ANATOMISCHE ZIELPOSITION
AGENCEMENT MÉDICAL POUR INTRODUIRE UN OBJET DANS UNE POSITION ANATOMIQUE CIBLE

(43) Date of publication of application: 13.01.2021
(73) Proprietor: HVR Cardio Oy, 02600 Espoo (FI)
(72) Inventor: KERÄNEN, Olli, 23741 Bjärred (SE)
(74) Representative: Berggren Oy

(56) References cited:
- WO-A2-2012/167095
- US-A1- 2005 107 812
- US-A1- 2010 185 172

## Description

### TECHNICAL FIELD OF THE INVENTION

The invention relates to a medical arrangement for introducing an object, such as an implant into an anatomical target position, such as a cardiac implant (like an annuloplasty medical device) into an annulus of a heart valve, such as a mitral valve or tricuspid valve.

### BACKGROUND

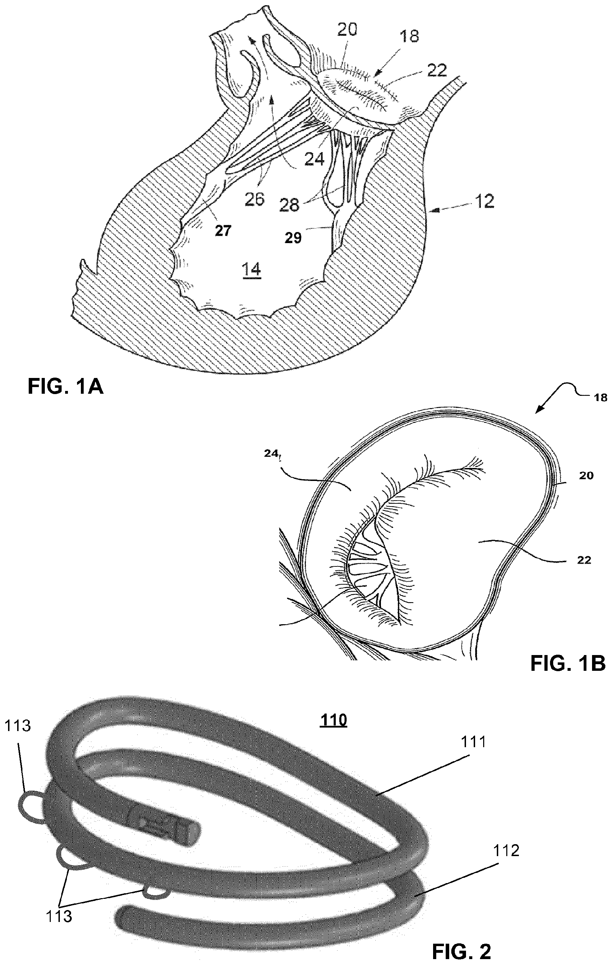

Fig. 1A illustrates a portion of the heart 12, the mitral valve 18, and the left ventricle 14 as an example of the anatomical target position. The mitral valve is at its boundary circumferenced by an annulus 20. The valve has two cusps or leaflets 22, 24. Each of these cusps or leaflets 22, 24 are connected to a respective papillary muscle 27, 29 via their respective connecting chordae 26, 28. In normal healthy individuals the free edges of the opposing leaflets will close the valve by coaptation. However, for some individuals the closure is not complete, which results in a regurgitation, also called valvular insufficiency, i.e. back flow of blood to the left atrium making the heart less effective and with potentially severe consequences for the patient. Fig. 1B illustrates a mitral valve 18, in which the leaflets 22, 24 do not close properly. This commonly occurs when the annulus 20 becomes dilated. One surgical procedure to correct this is to remove a portion of the leaflet 24 and stitch the cut edges together with one another. The procedure will pull back the annulus 20 to a more normal position. However the strength of the leaflet 24 is altered. Similar problems with a less effective heart function occur if one or both leaflets are perforated to such an extent that blood is flowing towards the left atrium, although the leaflets close properly.

In some conditions of degenerated heart function, the leaflets do not present a solid surface, as in a degenerative valve disease. The leaflet may also be ruptured, most commonly at an edge of a leaflet, resulting in an incomplete coaptation. Hence, cardiac devices and methods are developed for repairing of one or more leaflets of a heart valve, or other related anatomical structures, such as the chordae attached to the ventricular side of leaflets.

Figure 2 illustrates an exemplary implant to be delivered and introduced into an anatomical target position, and in particularly a cardiac implant 110. The implant may comprise one or more loop-shaped structures 111, 112. Advantageously one first loop-shaped structure is configured to abut a first side of the heart valve and one second loop-shaped structure is configured to abut a second, opposite, side of the valve to thereby trap a portion of the valve tissue 20 between the second and the first support structures 111, 112.

The implant is typically delivered via a catheter and has thus typically a delivery state, where the implant has an elongated form. In said delivery state the implant can be transferred advantageously through a catheter having diameter 7-10 mm, for example. The implant comprises typically a shape memory material having a first shape, such as the elongated form of the delivery state in a first temperature, and the second shape, such as the loop-shaped form in a second temperature. The second temperature corresponds advantageously essentially the body temperature, whereupon the implant takes the second shape, corresponding the loop-shaped form, when introduced for example with the blood flow in the atrium.

In addition, some problems arise due to a catheter system having both inner and outer catheters, usually steerable catheters, sometimes numerous inner catheters, where the inner catheter(s) locating inside the outer catheter limit(s) space from the implant.

It is found that the prior art cardiac implants, such as depicted above, work very well, but there are still some disadvantages relating to the introduction devices, such as catheter type devices, to deliver the implant into the anatomical target position, such as into the annulus of the heart valve. The catheter based systems are based for delivering a relative thick main catheter having a first curve portion into a first portion of the anatomical target position, such as to an atrium, then a second catheter having a second curve portion into a second portion of the anatomical target position, such as next to annulus or leaflets of the heart valve, and then a third catheter having a third curve portion into a third portion of the anatomical target position, such as around the annulus of the heart valve. In some systems there might be even further catheters to with further curve portions to be delivered before the implant can be delivered and introduced into its position. The third or further catheter if used, is called as a delivery catheter. The implant is then delivered to its position inside the feeding catheters, which is delivered to its position inside the other catheters.

In addition, a prior art document US 2010/185172 A1 discloses a solution for assessing an accessibility of a sub-valvular space of a heart and accessing the sub-valvular space of the heart and positioning one or more devices in the sub-valvular space of the heart. There, after diagnostic catheter has been positioned at a desired location in sub-annular groove region, a guidewire is advanced through a lumen of diagnostic catheter and partially routed around sub-annular groove region. After the guidewire has been positioned in sub-annular groove region, a guide tunnel may be advanced through guide catheter, over guidewire. After the guide tunnel has been positioned in the sub-annular groove region, the guidewire may be withdrawn proximally, and an anchor deployment catheter may then be advanced through the lumen of guide tunnel and toward opening at or adjacent to the distal tip of guide tunnel.

In addition, US 2005/107812 A1 discloses systems for facilitating positioning of a valve annulus treatment device. There, a first guide catheter may have a second, steerable guide catheter passed through/along the first guide catheter. A guide sheath may be passed over the second guide catheter, where the sheath may be a flexible, tubular member that may be passed over second guide catheter and within first guide catheter. Once the guide sheath is in place, the second guide catheter may be withdrawn. An anchor delivery device may then be advanced through the guide sheath.

There are some drawbacks related to the prior art catheter based systems, such as at least the main catheter must be thick (7-10 mm or even more) so that it can carry the further catheters inside. In addition, also the delivery catheter must be relative thick so that the implant can be delivered inside the delivery catheter. When the catheters are relatively thick, it is very hard to insert the catheters into the anatomical target position. For example, a sub-annular space below the annulus, so between the chordae and wall or septum, is very narrow, whereupon the best channel for the thick catheters is very difficult to find and deliver, in particularly when the maneuverability and steerability of the catheters is poor. Furthermore, the surface of the inner wall is rough, having additionally numerous attachment points of chordae, which easily causes stuck of the catheters. Additional challenges arise when the catheters, especially also the delivery catheter, have memory properties or predetermined shapes, which might activate too early and thereby raising possibility to stuck the catheter into the wall or other structures of the anatomical target position, such as the heart. Thus, the time limit to insert the catheters, in particularly the delivery catheter, having memory properties, is very limited so that the catheters can be inserted into their right and accurate position before the memory property will be activated by the temperature of the anatomical target position, such as the heart.

### SUMMARY

It is an object of the invention to alleviate and eliminate the problems relating to the known prior art. Especially the object of the invention is to provide a medical arrangement for introducing an implant into an anatomical target position in an easy, fast, safe and accurate manner with a high degree of control. In addition, the object of the invention is to minimize the sizes of the catheters used and at the same time minimize stuck of the catheters and thereby minimize stress introduced for the anatomical target position.

The object of the invention can be achieved by the features of independent claim.

The invention relates to a medical arrangement for introducing an implant into an anatomical target position into an annulus of a heart valve, according to claim 1 _

A medical arrangement according to the invention is configured to introduce an implant, and in particularly such as a cardiac implant, or an annuloplasty medical device, from a distal end of the arrangement into an anatomical target position, into an annulus of a heart valve. The heart valve may be a mitral valve or tricuspid valve, for example, not limiting to those only.

According to an example the object is the implant, which comprises in a use a loop shaped support portion, having either one or more loops or coils so that at least one first loop-shaped structure can be configured to abut a first side of the heart valve and at least one second loop-shaped structure to abut a second, opposite, side of the valve to thereby trap a portion of the valve tissue between the second and the first support structures. It is also possible that there is only the one first loop-shaped structure, which is configured to abut a first side of the heart valve, and not the second support structures, or vice versa. The implant is advantageously adapted to support for a mitral valve upon being fully delivered.

According to an embodiment the medical arrangement comprises a first introducer, such as an outer steerable introducer or catheter, and a guide wire. The first introducer is advantageously an outer steerable catheter, which is delivered for example to the atrium or ventricle, but not to the anatomical target end position as such, where the implant is to be introduced. According to an embodiment the guide wire is configured to be introduced into or towards said anatomical target position via the first introducer but before the implant (meaning that the guide wire is delivered beforehand so that it can guide the delivery of implant). When the guide wire is delivered into the right position, the implant is then delivered along and guided by the guide wire (and inside the first introducer) into the anatomical target position.

The guide wire can be also introduced partly from the distal end portion of the first introducer, after which also the implant can be delivered partly along the guide wire (i.e. not to a final end target position at once but rather step by step), and after which the guide wire may be further delivered and followed by the implant and thereby introducing the guide wire and implant sequentially. According to an embodiment the guide wire and implant can be delivered at the same time, but however so that the guide wire guides the route to the anatomical target position.

The implant is advantageously a hollow tubular structure, whereupon the implant can be delivered into said anatomical target position so that it travels around or over the guide wire. This offers clear advantages over the known prior art solutions namely because the guide wire is very thin compared to the catheters, it is remarkable easy, fast, safe and accurate to insert via very small and narrow channels of the anatomical target position to the correct place. When the guide wire is delivered to the correct position, the implant with the hollow tubular structure can be easily delivered along the guide wire to the correct end position. Alternatively, the implant may also have loops, stitches or turns coupled with it whereupon the implant, even a solid implant, can be delivered to the position so that the loops or turns travel around the guide wire. In addition, it is to be noted that the implant can be also made from fabric, polyurethane or polyester and there is no need for the implant to have any memory property. The implant can be delivered to the position for example by a pusher coupled to the proximal end of the implant, whereupon the implant can be pushed to the position by the pusher. In the case of a non-rigid implant, for example if the implant is made of textile or the like compressible material, the pusher can be coupled to the distal end of the implant and thereby to draw the implant to the position.

According to an embodiment the implant may have at least a first curved shape having a preformed shape and capable of being delivered in a straightened configuration through the first introducer, whereupon the implant is activated of guided to at least a first curved shape within or near the anatomical target position, such as in the atrium or ventricle proximate the mitral valve of the heart. The implant may comprise e.g. a shape memory property and biased to said curved shape. However, it is to be noted that according to the invention, in particularly the implants without any memory property can be delivered and guided by the guide wire, which is delivered to the position first.

It is to be noted that the implant is configured to follow said guide wire and said first curved shape of the guide wire into said anatomical target position. When the implant has been introduced into said anatomical target position, the guide wire can be retracted and the implant essentially maintains the shape taken when introduced into said anatomical target position, so the shape of the guide wire in the anatomical target position. It is to be noted that the implant can be attached, such as sutured, into its position for example by keeping the implant in its position during the attaching by the guide wire. The implant can also be as a self-attaching version having e.g. teeth, whereupon when the introducer over the implant is retracted the teeth can dig into the tissue and thereby attach the implant into its position.

The guide wire is advantageously retracted only after the implant is secured. This makes the attaching of the implant very safe because the implant can be retracted away as long as the guide wire is in the anatomical target position. In addition it is to be noted that when the guide wire is inside the implant and in the anatomical target position, the position of the implant can still be adjusted by manipulating the guide wire, such as drag and/or turn the guide wire and thereby adjust the position or orientation of the implant (a whole packet of the guide wire and the implant), which is very advantageous in many situations. In addition, if something goes wrong, the implant can still be retracted away by the guide wire. After the implant is attached to the anatomical target position the guide wire can be retracted away from the anatomical target position.

The guide wire has advantageously an activated shape and an inactivated shape, wherein in said inactivated shape the guide wire can be delivered in a straightened configuration through the first introducer and in said activated shape the guide wire takes at least a first curved shape within or near the anatomical target position, such as in the atrium or ventricle or proximate the mitral valve. The guide wire advantageously has a preformed shape so to take the activated shape when introduced to the anatomical target position. According to an embodiment the guide wire is at least partially formed from a shape memory material and thereby operable to assume said activated shape when meet the temperature of the anatomical target position. The activated shape can be achieved also via another techniques known by the skilled person. When the guide wire is activated to said at least first curved shape and delivered to the anatomical target position (or at least towards the position), after which the implant is delivered along the guide wire to the anatomical target position, as is described elsewhere in this document.

In addition, the distal end of the guide wire may comprise a curvature tip portion, such as a J-shape, in order to allowing smooth delivery of the distal end of the guide wire and to prevent the distal end of the guide wire to get tangled in to tissue. The curvature tip portion may have a preformed shape or being at least partially formed from a shape memory material and thereby taking the shape of the curvature tip portion when introduced into the anatomical target position.

According to an embodiment the arrangement additionally comprises a second introducer, such as a catheter and in particularly an inner steerable catheter. The second introducer is arranged to be operable between the first introducer and the guide wire. The second introducer is configured to be introduced from the distal end portion of the first introducer. According to the invention, the second introducer is used for example to bypass the leaflets of the heart or other anatomical portion in or near the anatomical target position. However, according to an embodiment at least a distal portion of the second introducer is configured to be introduced from the distal end portion of the first introducer before the guide wire, and thereby guide or instruct the guide wire to bypass for example a certain anatomical portion, such as leaflets

When the second introducer is used, the implant is then be delivered inside the second introducer (and also inside the first introducer), and again along the guide wire, into or towards the anatomical target position after at least the distal portion of the second introducer is introduced from the distal end portion of the first introducer.

However, the second introducer is advantageously configured to be retracted after said guide wire is introduced into said anatomical target position and/or before said implant is introduced into said anatomical target position. This is not mandatory but by this more space can be provided for the implant.

In addition, according to an embodiment, the arrangement may additionally comprise also a third introducer, where said third introducer is arranged to be operable between the first introducer and the guide wire, and if the second introducer is used, also between the second introducer and the guide wire. The third introducer can be used as a delivering catheter for delivering the implant into the anatomical target position inside the third introducer but still around the guide wire. When the implant is delivered inside the third introducer, it additionally protects the anatomical portions from the implant. The third introducer is configured to be introduced from the distal end portion of the first introducer (and from the distal end portion of the second introducer, if used). It is to be noted that at least a distal portion of the third introducer is introduced from the distal end portion of the first introducer (and from the distal end portion of the second introducer, if used) only after the guide wire is introduced into or towards said anatomical target position.

The third introducer is advantageously a flexible catheter, which is configured to be delivered along the guide wire into or towards said anatomical target position after the guide wire is at least partially introduced from the distal end portion of the first introducer. Most advantageously the third introducer is delivered to the anatomical target position when the guide wire is fully delivered to the anatomical target position, in particularly when the third introducer is flexible. The third introducer can be used to protect the tissue and other anatomical portions in the anatomical target position from the implant during delivering the implant into the anatomical target position. It is to be noted that the implant is configured to be delivered inside the third introducer into or towards said anatomical target position. This can be done after at least the distal portion of the third introducer is introduced from the distal end portion of the first and second introducers into or towards said anatomical target position, but most advantageously when the distal end of the third introducer is delivered into the anatomical target position.

According to an embodiment the second introducer is retraced before the implant is delivered into the anatomical target position inside the third introducer. This is not mandatory but by this more space can be provided for the implant, and especially when the third introducer is flexible so that it can expand for the implant during delivering. In addition, the third introducer is configured to be retracted after the guide wire and implant have been introduced into said anatomical target position.

Still in addition, the arrangement may also comprise a cooling arrangement for cooling the guide wire for example during retracting the guide wire, but also in other phases. When the guide wire is cooled down it can be easily reshaped and for example retracted out essentially easily transformable state. The cooling arrangement may be arranged so that there is an inlet for the cooling agent, such as cool water, in the proximal end of the first, second and/or third introducer, whereupon the cooling agent can flow between the walls of the first, second and/or third introducer from the proximal ends towards the distal ends, for example. According to an embodiment the distal ends may have openings so that the cooling agent can flow out.

The present invention offers advantages over the known prior art, such as an easy, safe, precise and time saving manner to reliable delivering the object, such as an implant to the anatomical target position, like to the annulus of the valve. Still, the guide wire is very convenient to deliver to the target position, namely it is very thin compared to the catheters and thus it does not tangle to the tissue. When the guide wire is delivered into the target position, the subsequent catheters, even flexible catheters, can be delivered easily along and guided by the guide wire. Further, the guide wire can be used as a rescue device during securing the object, like the implant, namely if something goes wrong in securing, any device used for pulling out the implant can be delivered to the proximal end of the implant along and guided by the guide wire, because the guide wire can be kept in the target position as long as the implant is secured.

In addition, the present invention provides for a compact arrangement for delivering the object. The compact medical device allows minimally invasive procedure. Furthermore, when using the compact catheter-operated medical device, risks for having any medical drawbacks or symptoms are much lower than e.g. in the traditional open-heart operation. Also, the patient recovery process is much faster.

The exemplary embodiments presented in this text are not to be interpreted to pose limitations to the applicability of the appended claims. The verb "to comprise" is used in this text as an open limitation that does not exclude the existence of also un-recited features. The features recited in depending claims are mutually freely combinable unless otherwise explicitly stated.

The novel features which are considered as characteristic of the invention are set forth in particular in the appended claims. The invention itself, however, both as to its construction and its method of operation, together with additional objects and advantages thereof, will be best understood from the following description of specific example embodiments when read in connection with the accompanying drawings.

### BRIEF DESCRIPTION OF THE DRAWINGS

Next the invention will be described in greater detail with reference to exemplary embodiments in accordance with the accompanying drawings, in which:
- Figures 1A-1B: illustrate schematically a portion of a heart and mitral valve,
- Figure 2: illustrates an exemplary tissue anchoring unit for securing the flexible leaflet to the adjacent tissue and/or annulus according to an advantageous embodiment of the invention, and
- Figures 3-23: illustrate a medical arrangement for introducing an implant into an anatomical target position according to advantageous embodiments of the invention.

### DETAILED DESCRIPTION

Figures 1A-1B and 2 are already discussed in more details in connection with the background of the invention portion above.

In addition, it is to be noted that the implant 110 comprises advantageously a hollow structure so that it can be passed along the guide wire 103 so that the guide wire 103 is inside the hollow structured implant 110. However, the implant 110 may also have loops, stitches or turns 113 coupled with it, as can be seen in Figure 2, whereupon the implant, even a solid implant, can be delivered to the position so that the loops or turns 113 travel around the guide wire 103.

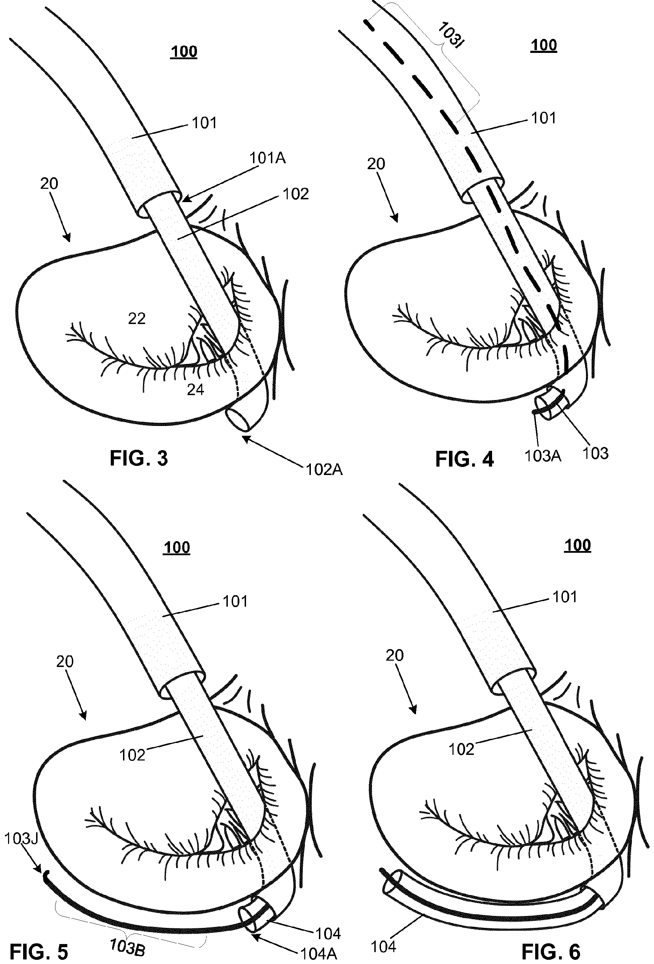

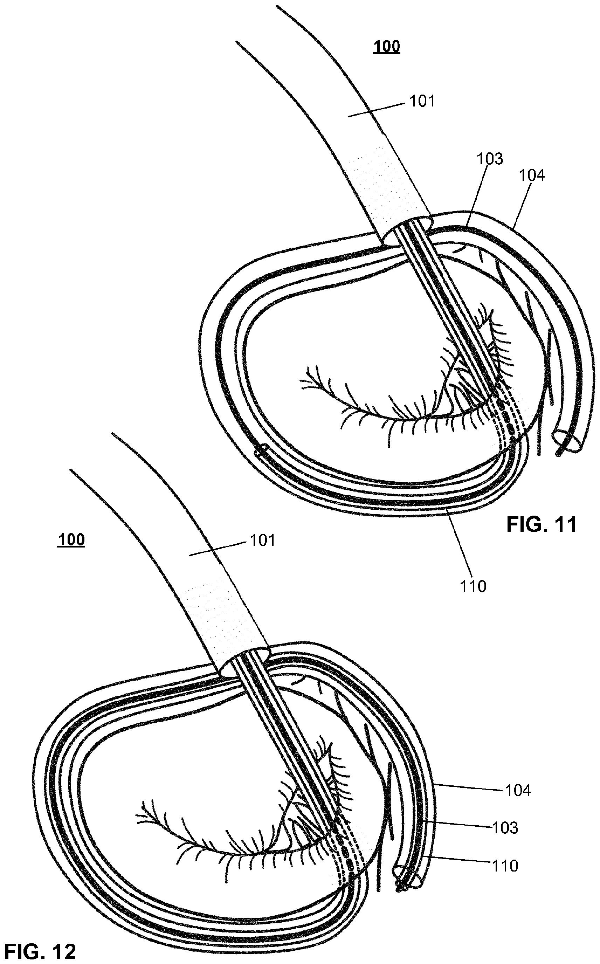

Figures 3-23 illustrate a medical arrangement 100 for introducing an implant into a heart as an example of the anatomical target position 20 according to advantageous embodiments of the invention. In Fig 3 the first introducer 101 is delivered next to the annulus 20 of the heart, and the second introducer 102 is introduced from the distal end portion 101A of the first introducer 101. It is to be noted that the second introducer is an optional introducer, but it is still illustrated in Figures as an example. The second introducer can be used to bypass the leaflets 22, 24 of the heart, as well as to achieve a turn to a specific direction by turning the distal end 102A of the second introducer 102 (namely turnability of the introducers are limited). In addition, it is to be noted that at least a distal portion 102A of the second introducer 102 is introduced from the distal end portion 101A of the first introducer 101 before the guide wire, and thereby guide or instruct the guide wire 103 to bypass the leaflets, for example, as can be seen in Fig 4.

When the second introducer has bypassed the leaflets 22, 24, the guide wire 103 can be delivered into or towards the position 20. It is to be noted that the guide wire 103 is delivered into the position before the implant 110.

The guide wire 103 has advantageously an activated shape and an inactivated shape, wherein in said inactivated shape the guide wire 103 can be delivered in a straightened configuration 103I through the first introducer 101, as can be seen in Fig. 4. In said activated shape the guide wire 103 takes at least a first curved shape 103B within or near the anatomical target position 20. When the guide wire 103 is activated to said at least first curved shape 103B, it is delivered to or a least towards the position. According to an example the distal end 103A of the guide wire 103 may comprise a curvature tip portion 103J, such as a J-shape, in order to allowing smooth delivery of the distal end 103A of the guide wire 103 and to prevent the distal end 103A of the guide wire 103 to get tangled in to tissue.

The arrangement may additionally comprise also a third introducer 104, as can be seen in Figs. 5-11, where the third introducer 104 is operated between the first introducer 101 and the guide wire 103, and if the second introducer is used, also between the second introducer 102 and the guide wire 103. The third introducer 104 is introduced from the distal end portion 101A of the first introducer 101 (and from the distal end portion 102A of the second introducer 102, if used). It is to be noted that at least a distal portion 104A of the third introducer 104 is introduced from the distal end portion 101A of the first introducer 101 (and from the distal end portion 102A of the second introducer 102, if used) only after the guide wire 103 is introduced into or towards said anatomical target position 20.

The third introducer 104 is delivered along the guide wire 103 into or towards said anatomical target position 20 after the guide wire 103 is at least partially introduced from the distal end portion 101A of the first introducer 101, as can be seen in Figs. 5-8. Most advantageously the third introducer 104 is delivered to the anatomical target position 20 when the guide wire is fully delivered to the anatomical target position, in particularly when the third introducer 104 is flexible, whereupon the third introducer 104 is guided by the guide wire 103.

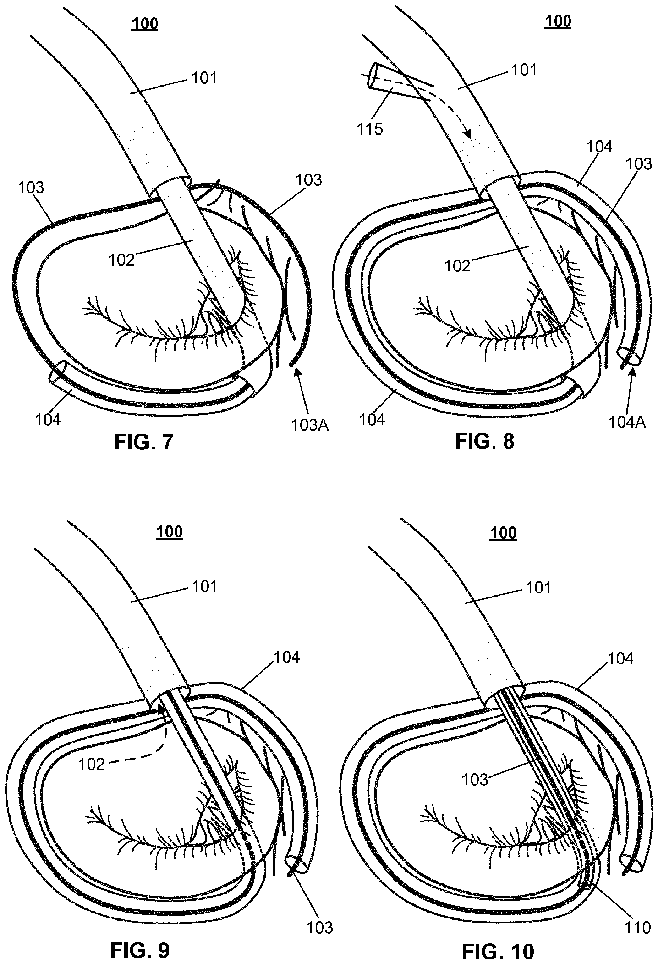

When the guide wire 103 and the third introducer 104 are delivered into the position, the second introducer 102 is retracted and it is retracted advantageously before delivering the implant 110 into the anatomical target position 20, as is the case in Fig. 9. This is not mandatory but by this a more space can be arranged for the implant. It is to be noted that also the first introducer can be retracted before delivering the implant 110 or even right after the delivering of the second introducers, even if this is not shown in the Figures. By this even more space can be arranged for the implant.

The implant 110 is then delivered around (and guided by) the guide wire 103 and inside the third introducer 104 into or towards said anatomical target position, as can be seen in Figures 10-12. This can be done after at least the distal portion 104A of the third introducer 104 is introduced from the distal end portion 101A of the first and second introducers 101, 102 into or towards said anatomical target position, but most advantageously when the distal end of the third introducer 104 is delivered into the anatomical target position.

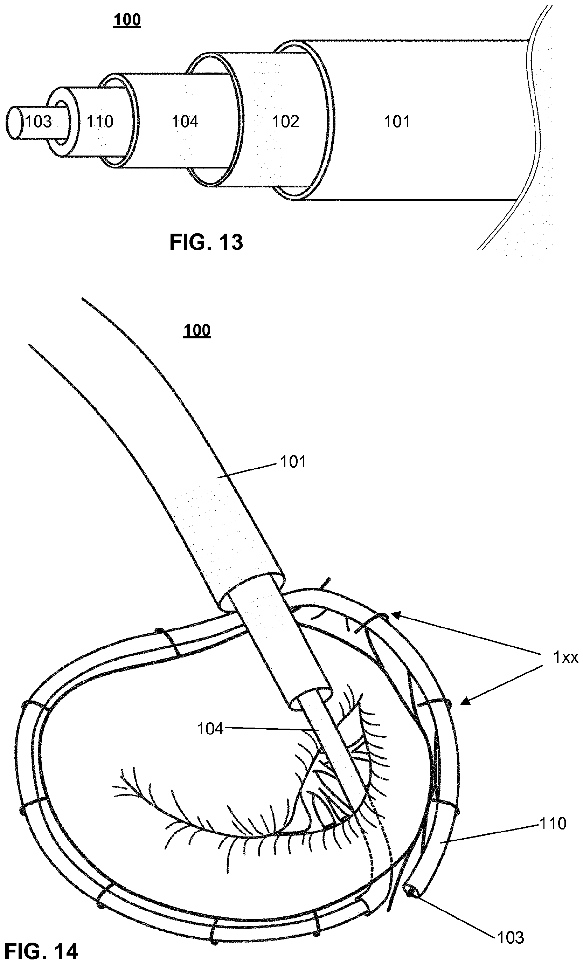

The third introducer 104 is then retracted after the implant 110 is introduced into the anatomical target position 20 after which the implant can be secured to the tissue by securing members 114, as can be seen in Fig. 14. The securing can be done for example by suturing or stabling or by other securing methods known by the skilled person. In addition, the third introducer 104 can be retracted sequentially uncovering only a part of the implant for securing and after securing said uncovered part the third introducer 104 can be retracted more, thereby uncovering additional portion of the implant for securing.

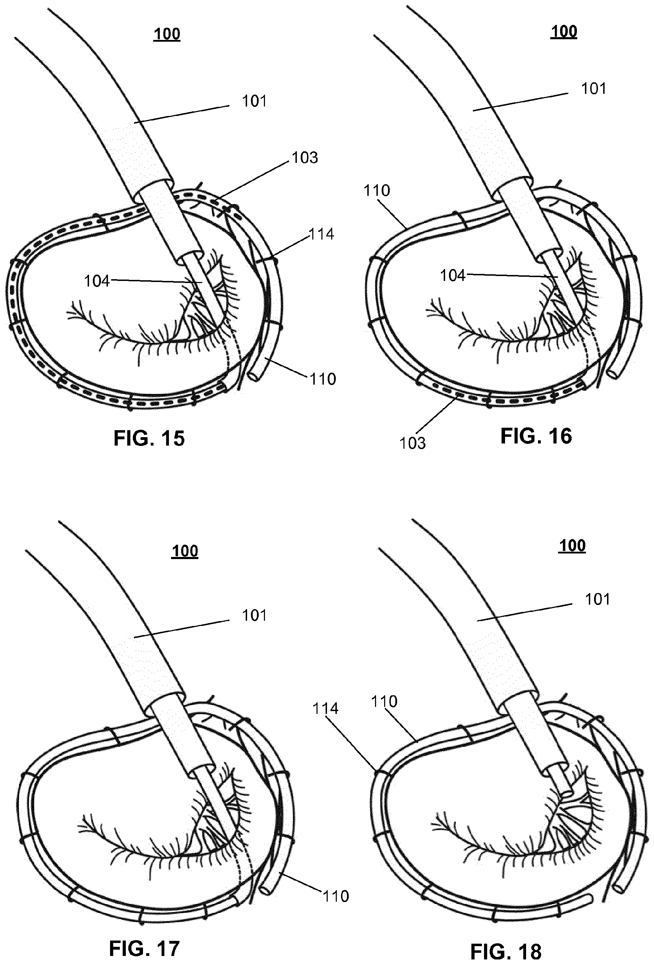

When the implant 110 is introduced and secured into the position 20 (and also the third introduces is retracted), as is the case in Fig. 10, the guide wire 103 can be retracted and the implant 110 essentially maintains the shape taken when introduced into said anatomical target position 20, so the shape of the guide wire in the anatomical target position, as can be seen in Figs. 15-18.

Figure 13 illustrates the order of the introducers 101, 102 104, guide wire 103 and the implant 110.

In addition, Fig. 8 illustrates also a cooling arrangement 115 for supplying the cooling agent (arrow) and thereby cooling the guide wire 103. It is to be noted that the arrangement may comprise one or more cooling arrangements 115 and arranged in connection with the first, second and/or third introducers. In addition, even if the cooling arrangement 115 is illustrated only in connection with Fig. 8, it should be understood, that it may also be comprised by other embodiments and arrangements 100 illustrated in other Figures.

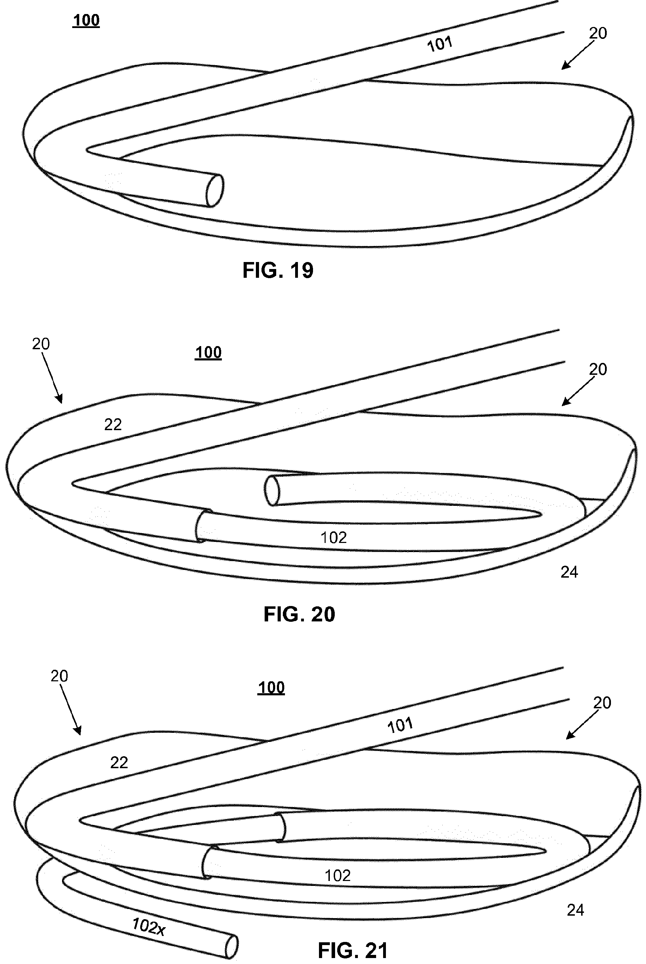

Figures 19-23 illustrate a further example of the medical arrangement 100 to introduce the implant 110 into a mitral valve 20 (as the anatomical target position) so that at least one loop-shaped structure 111 of the implant 110 abuts a first side of the heart valve and one second loop-shaped structure 112 abuts a second, opposite, side of the valve to thereby trap a portion of the valve tissue 20 between the second and the first support structures 111, 112. In Figs. 19-23 the first introducer 110 has a first curve shape and the second introducer 102 has a second curve shape to the same curvature direction as the first curve shape of the first introducer 101 so to form a concentric system 100. As can be seen in Fig. 21, the arrangement may also have an additional second introducer 102x, which again has third curve shape but still to the same curvature direction as the first and second curve shapes of the first and second introducers, wherein said first, second and third curved shapes are concentric curved shapes. In addition, the additional second introducer 102x may also be a steerable catheter or having pre-curved structure so that it has ability to seek said third curve shape at least and advantageously when delivered into or towards the anatomical target position.

It is to be noted that the first introducer is introduced next to the annulus and the second as well as additional second introducers 102, 102x are used to bypass the leaflets 22, 24 and to be introduced to the opposite side of the annulus as the first introducer is delivered.

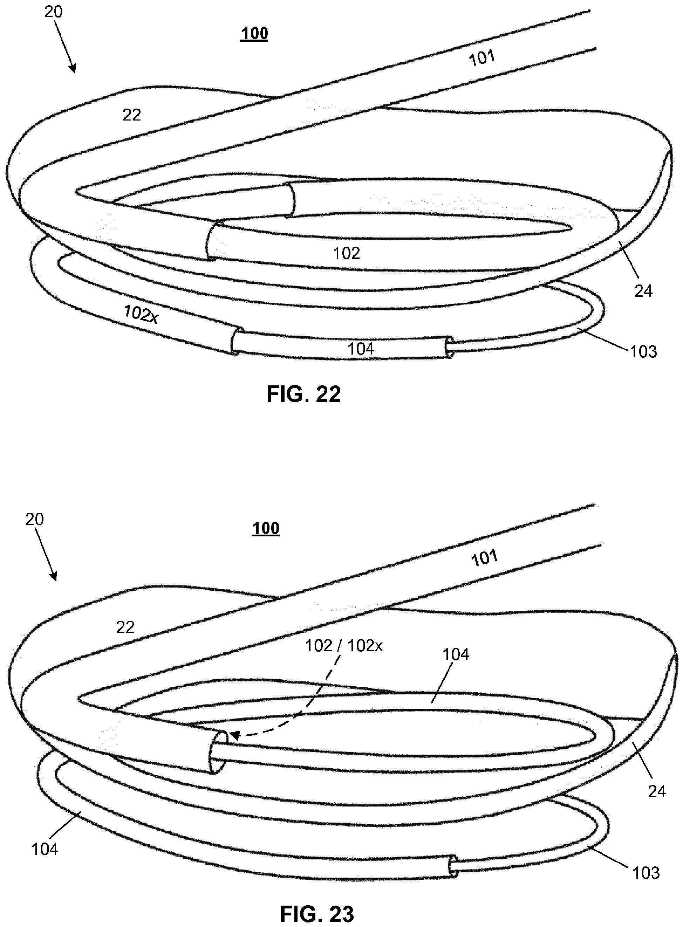

Figures 22 and 23 illustrate the arrangement 100, where the third introducer 104 and also the guide wire 103 are introducer into or at least towards the position for delivering the implant. As can be seen in Fig. 23, the second as well as additional second introducers 102, 102x can be retracted before introducing the implant. The implant and the steps for delivering it are not shown, but the fundamental principles are the same as described elsewhere in this document.

It is to be noted that according to an embodiment the first introducer 101 can be retracted already after the second introducer 102 is delivered and before the delivery of the other introducers 102x, 104 and implant, and the second introducer 102 can be retracted after the additional second introducer 102x is delivered and before the delivery of the third introducer 104 and implant, and that the additional second introducer 102x can be retracted after the third introducer 104 is delivered toward or into the anatomical target position and before the delivery of the implant 110. In this way a maximum space can be provided for the delivering catheter 104, or the third introducer 104, and in particularly when the third introducer 104 in an expandable introducer 104, whereupon relatively big implant can be delivered into the anatomical target position. Previously, the diameter of the all additional introducers must have been smaller and smaller, whereupon the diameter of the last delivering catheter is particularly small, this remarkably limiting also the size of the object, such as the implant, to be delivered.

The disclosure has been explained above with reference to the aforementioned embodiments, and several advantages of the invention have been demonstrated.

For example the guide wire is at least partially formed from a shape memory material operable to assume an activated shape and an inactivated shape, wherein in said inactivated shape the guide wire is configured to be delivered in a straightened configuration through said first introducer and in said activated shape the guide wire is configured to take said at least first curved shape within or near the anatomical target position. The guide wire is advantageously configured to be introduced before the implant into or towards the anatomical target position. However, it is to be noted that the guide wire should at least reach the target position before the implant.

In addition, it is to be noted that even if the implant is described in this document as an example to be delivered, also other kinds of object can be delivered such as medicaments, for example, Furthermore, even if the heart is described in many embodiments, it is to be understood that the heart is only an example of the anatomical target. Still, in addition it is to be noted that the implant can be rigid or flexible.

## Claims

1. A medical arrangement (100) configured to introduce an implant (110) from a distal end of the arrangement into an anatomical target position (20) around an annulus of a mitral valve (18) of a heart, between an atrium and ventricle (14), the medical arrangement comprising a first introducer (101) and a guide wire (103),
- said guide wire (103) has at least a first curved shape,
∘ said at least first curved shape having a preformed shape capable of being delivered in a straightened configuration (103I) through said first introducer (101) and
∘ configured to be activated to said at least first curved shape (103B) within or near the anatomical target position (20),
**characterized in that**
- the arrangement additionally comprises a second introducer (102) for bypassing the leaflets, where said second introducer (102) is arranged to be operable between the first introducer (101) and the guide wire (103),
∘ wherein the second introducer (102) is configured to be introduced from the distal end portion (101A) of the first introducer (101) and wherein at least a distal portion (102A) of the second introducer (102) is configured to be introduced from the distal end portion (101A) of the first introducer (101) before the guide wire, and
∘ wherein the second introducer and the first introducer (101) have curved shapes forming a concentric system,
- said guide wire (103) is configured to be introduced from the atrium and through the first introducer to bypass the leaflets through the second introducer to the ventricle before the implant (110),
- said implant (110) is configured to be delivered along the guide wire (103) around the annulus (20) after the guide wire (103) is at least partially introduced from the distal end portion (101A) of the first introducer (101), whereupon said implant (110) is configured to follow said guide wire (103) and said first curved shape (103B) of the guide wire around the annulus (20), wherein said implant (110) is configured to be delivered inside the second introducer (102) into or towards said anatomical target position (20) after at least the distal portion (102A) of the second introducer (102) is introduced from the distal end portion (101A) of the first introducer (101), and
- said guide wire (103) is configured to be retracted after said implant (110) has been introduced around the annulus (20) so that said implant (110) essentially maintains the shape taken when introduced into said anatomical target position (20) and can be attached to the annulus (20).

2. An arrangement of claim 1, wherein said implant (110) is configured to be delivered along the guide wire (103) into said anatomical target position (20) after the guide wire (103) is at least partially introduced from the distal end portion (101A) of the first introducer (101) and when the guide wire (103) is activated to said at least first curved shape (103B).

3. An arrangement of claim 1 or 2, wherein the second introducer (102) is a catheter, in particularly a steerable catheter (102), and wherein said first introducer (101) is a catheter, in particularly an outer steerable catheter (101).

4. An arrangement of any of claims 1-3, wherein said second introducer (102) is configured to be retracted after said guide wire (103) is introduced into said anatomical target position (20) and/or before said implant (110) is introduced into said anatomical target position (20).

5. An arrangement of any previous claims, wherein the arrangement additionally comprises a third introducer (104), where said third introducer (104) is arranged to be operable between the first introducer (101) and the guide wire (103), and between the second introducer (102) and the guide wire (103).

6. An arrangement of claim 5, wherein the third introducer (104) is configured to be introduced from the distal end portion (101A) of the first introducer (101) and wherein at least a distal portion (104A) of the third introducer (104) is configured to be introduced from the distal end portion (101A) of the first introducer (101) after the guide wire (103) is introduced into or towards said anatomical target position (20).

7. An arrangement of claim 6, wherein the third introducer (104) is configured to be introduced inside or from the distal end portion (102A) of the second introducer (102) when the second introducer (102) is first delivered to its target position and wherein said second introducer (102) is configured to be retracted before said implant (110) is introduced into said anatomical target position (20).

8. An arrangement of any of claims 5-6, wherein the third introducer (104) is a flexible catheter, which is configured to be delivered along the guide wire (103) into or towards said anatomical target position (20) after the guide wire (103) is at least partially introduced from the distal end portion (101A) of the first introducer (101).

9. An arrangement of any of claims 5-7, wherein said implant (110) is configured to be delivered inside the third introducer (104) into or towards said anatomical target position (20) after at least the distal portion (104A) of the third introducer (104) is introduced from the distal end portion (101A) of the first and/or second introducers (101, 102) into or towards said anatomical target position (20).

10. An arrangement of any of claims 5-9, wherein said third introducer (104) is configured to be retracted after the implant (110) has been introduced into said anatomical target position (20) and wherein the implant (110) is configured to be secured to the anatomical target position after the third introducer (104) is retracted.

11. An arrangement of any of previous claims, wherein the distal end (103A) of the guide wire (103) comprises a curvature tip portion (103J), such as a J-shape, in order to prevent the distal end (103A) of the guide wire (103) to get tangled in to tissue.

12. An arrangement of any of previous claims, wherein said implant comprises a loop shaped support portion, having one or more loops or coils (111, 112) and adapted to support said anatomical target position, such as a mitral valve, upon being fully delivered, and wherein said implant comprises a hollow tubular structure or loop, stich or turn structure (113) attached to the implant, whereupon the implant (110) is configured to be delivered into said anatomical target position (20) so that it travels around said guide wire (110).

## Patentansprüche

1. Medizinische Anordnung (100), die dazu konfiguriert ist, ein Implantat (110) von einem distalen Ende der Anordnung in eine anatomische Zielposition (20) um einen Annulus einer Mitralklappe (18) eines Herzens zwischen einem Atrium und einem Ventrikel (14) einzuführen, wobei die medizinische Anordnung einen ersten Einführer (101) und einen Führungsdraht (103) umfasst,
- der Führungsdraht (103) mindestens eine erste gekrümmte Form aufweist,
∘ die mindestens erste gekrümmte Form eine vorgeformte Form aufweist, die in einer begradigten Konfiguration (103I) durch den ersten Einführer (101) zugeführt werden kann und
∘ so konfiguriert ist, dass sie aktiviert werden kann, um die mindestens erste gekrümmte Form (103B) innerhalb oder nahe der anatomischen Zielposition (20) einzunehmen, **dadurch gekennzeichnet, dass**
- die Anordnung zusätzlich einen zweiten Einführer (102) zum Umgehen der Klappen umfasst, wobei der zweite Einführer (102) so angeordnet ist, dass er zwischen dem ersten Einführer (101) und dem Führungsdraht (103) bedienbar ist,
∘ wobei der zweite Einführer (102) so konfiguriert ist, dass er vom distalen Endabschnitt (101A) des ersten Einführers (101) aus eingeführt werden kann, und wobei mindestens ein distaler Abschnitt (102A) des zweiten Einführers (102) so konfiguriert ist, dass er vom distalen Endabschnitt (101A) des ersten Einführers (101) aus vor dem Führungsdraht eingeführt werden kann, und
∘ wobei der zweite Einführer und der erste Einführer (101) gekrümmte Formen aufweisen, die ein konzentrisches System bilden,
- der Führungsdraht (103) so konfiguriert ist, dass er vom Atrium und durch den ersten Einführer eingeführt werden kann, um die Klappen durch den zweiten Einführer vor dem Implantat (110) in den Ventrikel zu umgehen,
- das Implantat (110) so konfiguriert ist, dass es entlang des Führungsdrahts (103) um den Anulus (20) herumgeführt wird, nachdem der Führungsdraht (103) zumindest teilweise vom distalen Endabschnitt (101A) des ersten Einführers (101) eingeführt wurde, wobei das Implantat (110) so konfiguriert ist, dass es dem Führungsdraht (103) und der ersten gekrümmten Form (103B) des Führungsdrahts um den Anulus (20) herum folgt, wobei das Implantat (110) so konfiguriert ist, dass es innerhalb des zweiten Einführers (102) in Richtung oder zu der anatomischen Zielposition (20) geführt wird, nachdem zumindest der distale Abschnitt (102A) des zweiten Einführers (102) vom distalen Endabschnitt (101A) des ersten Einführers (101) eingeführt wurde, und
- der Führungsdraht (103) so konfiguriert ist, dass er zurückgezogen werden kann, nachdem das Implantat (110) um den Anulus (20) herum eingeführt wurde, so dass das Implantat (110) im Wesentlichen die Form beibehält, die es beim Einführen in die anatomische Zielposition (20) angenommen hat, und am Anulus (20) befestigt werden kann.

2. Anordnung nach Anspruch 1, wobei das Implantat (110) so konfiguriert ist, dass es entlang des Führungsdrahts (103) in die anatomische Zielposition (20) eingeführt wird, nachdem der Führungsdraht (103) zumindest teilweise vom distalen Endabschnitt (101A) des ersten Einführers (101) eingeführt wurde und wenn der Führungsdraht (103) aktiviert wird, um zumindest die erste gekrümmte Form (103B) anzunehmen.

3. Anordnung nach Anspruch 1 oder 2, wobei der zweite Einführer (102) ein Katheter, insbesondere ein lenkbarer Katheter (102), ist, und wobei der erste Einführer (101) ein Katheter, insbesondere ein äußerer lenkbarer Katheter (101), ist.

4. Anordnung nach einem der Ansprüche 1 bis 3, wobei der zweite Einführer (102) so konfiguriert ist, dass er zurückgezogen werden kann, nachdem der Führungsdraht (103) in die anatomische Zielposition (20) eingeführt wird und/oder bevor das Implantat (110) in die anatomische Zielposition (20) eingeführt wird.

5. Anordnung nach einem der vorhergehenden Ansprüche, wobei die Anordnung zusätzlich einen dritten Einführer (104) umfasst, wobei der dritte Einführer (104) so angeordnet ist, dass er zwischen dem ersten Einführer (101) und dem Führungsdraht (103) und zwischen dem zweiten Einführer (102) und dem Führungsdraht (103) bedienbar ist.

6. Anordnung nach Anspruch 5, wobei der dritte Einführer (104) so konfiguriert ist, dass er vom distalen Endabschnitt (101A) des ersten Einführers (101) aus eingeführt werden kann, und wobei zumindest ein distaler Abschnitt (104A) des dritten Einführers (104) so konfiguriert ist, dass er vom distalen Endabschnitt (101A) des ersten Einführers (101) aus eingeführt werden kann, nachdem der Führungsdraht (103) in Richtung oder zu der anatomischen Zielposition (20) eingeführt wurde.

7. Anordnung nach Anspruch 6, wobei der dritte Einführer (104) so konfiguriert ist, dass er in oder aus dem distalen Endabschnitt (102A) des zweiten Einführers (102) eingeführt wird, wenn der zweite Einführer (102) zum ersten Mal in seine Zielposition gebracht wird, und wobei der zweite Einführer (102) so konfiguriert ist, dass er zurückgezogen wird, bevor das Implantat (110) in die anatomische Zielposition (20) eingeführt wird.

8. Anordnung nach einem der Ansprüche 5 bis 6, wobei der dritte Einführer (104) ein flexibler Katheter ist, der so konfiguriert ist, dass er entlang des Führungsdrahts (103) in Richtung oder zu der anatomischen Zielposition (20) geführt wird, nachdem der Führungsdraht (103) zumindest teilweise vom distalen Endabschnitt (101A) des ersten Einführers (101) eingeführt wurde.

9. Anordnung nach einem der Ansprüche 5 bis 7, wobei das Implantat (110) so konfiguriert ist, dass es innerhalb des dritten Einführers (104) in Richtung oder zu der anatomischen Zielposition (20) eingeführt wird, nachdem zumindest der distale Abschnitt (104A) des dritten Einführers (104) vom distalen Endabschnitt (101A) des ersten und/oder zweiten Einführers (101, 102) in Richtung oder zu der anatomischen Zielposition (20) eingeführt wurde.

10. Anordnung nach einem der Ansprüche 5 bis 9, wobei der dritte Einführer (104) so konfiguriert ist, dass er zurückgezogen wird, nachdem das Implantat (110) in die anatomische Zielposition (20) eingeführt wurde, und wobei das Implantat (110) so konfiguriert ist, dass es an der anatomischen Zielposition befestigt wird, nachdem der dritte Einführer (104) zurückgezogen wurde.

11. Anordnung nach einem der vorhergehenden Ansprüche, wobei das distale Ende (103A) des Führungsdrahtes (103) einen gekrümmten Spitzenabschnitt (103J), beispielsweise eine J-Form, aufweist, um zu verhindern, dass sich das distale Ende (103A) des Führungsdrahtes (103) im Gewebe verfängt.

12. Anordnung nach einem der vorhergehenden Ansprüche, wobei das Implantat einen schleifenförmigen Stützabschnitt mit einer oder mehreren Schleifen oder Spulen (111, 112) umfasst und so angepasst ist, dass er die anatomische Zielposition, wie etwa eine Mitralklappe, nach vollständiger Einbringung stützt, und wobei das Implantat eine hohle röhrenförmige Struktur oder Schleifen-, Stich- oder Wendestruktur (113) umfasst, die an dem Implantat befestigt ist, wobei das Implantat (110) so konfiguriert ist, dass es in die anatomische Zielposition (20) eingebracht wird, so dass es um den Führungsdraht (110) herumläuft.

## Revendications

1. Agencement médical (100) configuré pour introduire un implant (110) depuis une extrémité distale de l'agencement dans une position cible anatomique (20) autour d'un anneau d'une valvule mitrale (18) d'un cœur, entre une oreillette et un ventricule (14), l'agencement médical comprenant un premier introducteur (101) et un fil guide (103),
- ledit fil guide (103) a au moins une première forme incurvée,
∘ ladite au moins première forme incurvée ayant une forme préformée capable d'être délivrée dans une configuration redressée (103I) à travers ledit premier introducteur (101) et
∘ configuré pour être activé selon ladite au moins première forme incurvée (103B) à l'intérieur ou à proximité de la position cible anatomique (20), **caractérisé en ce que**
- l'agencement comprend en plus un deuxième introducteur (102) pour contourner les feuillets, où ledit deuxième introducteur (102) est agencé pour pouvoir fonctionner entre le premier introducteur (101) et le fil guide (103),
∘ dans lequel le deuxième introducteur (102) est configuré pour être introduit depuis la partie d'extrémité distale (101A) du premier introducteur (101) et dans lequel au moins une partie distale (102A) du deuxième introducteur (102) est configurée pour être introduite depuis la partie d'extrémité distale (101A) du premier introducteur (101) avant le fil guide, et
∘ dans lequel le deuxième introducteur et le premier introducteur (101) ont des formes incurvées formant un système concentrique,
- ledit fil guide (103) est configuré pour être introduit depuis l'oreillette et à travers le premier introducteur pour contourner les feuillets à travers le deuxième introducteur jusqu'au ventricule avant l'implant (110),
- ledit implant (110) est configuré pour être délivré le long du fil guide (103) autour de l'anneau (20) après que le fil guide (103) a été au moins partiellement introduit depuis la partie d'extrémité distale (101A) du premier introducteur (101), après quoi ledit implant (110) est configuré pour suivre ledit fil guide (103) et ladite première forme incurvée (103B) du fil guide autour de l'anneau (20), dans lequel ledit implant (110) est configuré pour être délivré à l'intérieur du deuxième introducteur (102) dans ou vers ladite position cible anatomique (20) après qu'au moins la partie distale (102A) du deuxième introducteur (102) a été introduite depuis la partie d'extrémité distale (101A) du premier introducteur (101), et
- ledit fil guide (103) est configuré pour être rétracté après que ledit implant (110) a été introduit autour de l'anneau (20) de sorte que ledit implant (110) conserve essentiellement la forme prise lorsqu'il est introduit dans ladite position cible anatomique (20) et peut être fixé à l'anneau (20).

2. Agencement selon la revendication 1, dans lequel ledit implant (110) est configuré pour être délivré le long du fil guide (103) dans ladite position cible anatomique (20) après que le fil guide (103) a été au moins partiellement introduit depuis la partie d'extrémité distale (101A) du premier introducteur (101) et lorsque le fil guide (103) est activé selon ladite au moins première forme incurvée (103B).

3. Agencement selon la revendication 1 ou 2, dans lequel le deuxième introducteur (102) est un cathéter, en particulier un cathéter orientable (102), et dans lequel ledit premier introducteur (101) est un cathéter, en particulier un cathéter orientable externe (101).

4. Agencement selon l'une quelconque des revendications 1 à 3, dans lequel ledit deuxième introducteur (102) est configuré pour être rétracté après que ledit fil guide (103) a été introduit dans ladite position cible anatomique (20) et/ou avant que ledit implant (110) ne soit introduit dans ladite position cible anatomique (20).

5. Agencement selon l'une quelconque des revendications précédentes, dans lequel l'agencement comprend en plus un troisième introducteur (104), où ledit troisième introducteur (104) est agencé pour pouvoir fonctionner entre le premier introducteur (101) et le fil guide (103), et entre le deuxième introducteur (102) et le fil guide (103).

6. Agencement selon la revendication 5, dans lequel le troisième introducteur (104) est configuré pour être introduit depuis la partie d'extrémité distale (101A) du premier introducteur (101) et dans lequel au moins une partie distale (104A) du troisième introducteur (104) est configurée pour être introduite depuis la partie d'extrémité distale (101A) du premier introducteur (101) après que le fil guide (103) a été introduit dans ou vers ladite position cible anatomique (20).

7. Agencement selon la revendication 6, dans lequel le troisième introducteur (104) est configuré pour être introduit à l'intérieur ou depuis la partie d'extrémité distale (102A) du deuxième introducteur (102) lorsque le deuxième introducteur (102) est d'abord amené à sa position cible et dans lequel ledit deuxième introducteur (102) est configuré pour être rétracté avant que ledit implant (110) ne soit introduit dans ladite position cible anatomique (20).

8. Agencement selon l'une quelconque des revendications 5 à 6, dans lequel le troisième introducteur (104) est un cathéter flexible, qui est configuré pour être délivré le long du fil guide (103) dans ou vers ladite position cible anatomique (20) après que le fil guide (130) a été au moins partiellement introduit depuis la partie d'extrémité distale (101A) du premier introducteur (101).

9. Agencement selon l'une quelconque des revendications 5 à 7, dans lequel ledit implant (110) est configuré pour être délivré à l'intérieur du troisième introducteur (104) dans ou vers ladite position cible anatomique (20) après qu'au moins la partie distale (104A) du troisième introducteur (104) a été introduite depuis la partie d'extrémité distale (101A) du premier et/ou du deuxième introducteur(s) (101, 102) dans ou vers ladite position cible anatomique (20).

10. Agencement selon l'une quelconque des revendications 5 à 9, dans lequel ledit troisième introducteur (104) est configuré pour être rétracté après que l'implant (110) a été introduit dans ladite position cible anatomique (20) et dans lequel l'implant (110) est configuré pour être fixé à la position cible anatomique après que le troisième introducteur (104) est rétracté.

11. Agencement selon l'une quelconque des revendications précédentes, dans lequel l'extrémité distale (103A) du fil guide (103) comprend une partie de pointe de courbure (103J), telle qu'une forme en J, afin d'empêcher l'extrémité distale (103A) du fil guide (103) de s'emmêler dans les tissus.

12. Agencement selon l'une quelconque des revendications précédentes, dans lequel ledit implant comprend une partie de support en forme de boucle, ayant une ou plusieurs boucles ou bobines (111, 112) et adaptée pour supporter ladite position cible anatomique, telle qu'une valvule mitrale, après avoir été entièrement délivrée, et dans lequel ledit implant comprend une structure tubulaire creuse ou une structure à boucle, couture ou tour (113) fixée à l'implant, après quoi l'implant (110) est configuré pour être délivré dans ladite position cible anatomique (20) de sorte qu'il se déplace autour dudit fil guide (110).
